# EUROPÄISCHE PATENTSCHRIFT

(11) **EP 1 070 740 B1**
(45) Veröffentlichungstag und Bekanntmachung des Hinweises auf die Patenterteilung: **02.01.2008**
(21) Anmeldenummer: 99114123.5
(22) Anmeldetag: 19.07.1999
(51) Int. Cl.: C08L 1/02, A61K 9/20, A61K 47/38

(54) **Coprozessiertes Polysaccharidprodukt mit unlöslicher Carboxymethylcellulose**
Coprocessed polysaccharide product with insoluble carboxymethylcellulose
Produit polysaccharidique co-travaillé avec une carboxyméthylcellulose insoluble

(43) Veröffentlichungstag der Anmeldung: 24.01.2001
(73) Patentinhaber: fit GmbH, 02788 Hirschfelde (DE)
(72) Erfinder: Bauer, Kurt H.,Prof. Dr., 79112 Freiburg (DE)
(74) Vertreter: Sommer, Peter

(56) Entgegenhaltungen:
- EP-A- 0 744 174
- WO-A-98/03064
- US-A- 3 539 365
- US-A- 5 769 934

## Beschreibung

Die Erfindung betrifft mit unlöslicher Carboxymethylcellulose als Supersprengmittel coprozessierte Reincelluloseprodukte, die sich einerseits durch gute Tablettiereigenschaften entsprechend ihrer hervorragenden Plastizität auszeichnen und die andererseits durch eine außerordentlich schnelle und starke Flüssigkeitsaufnahme die Quellung der enthaltenen Supersprengmittel beim Einbringen in Wasser oder wasserhaltige Flüssigkeiten rasch zerfallen. Dieser rasche Zerfall wird durch die innige Vermischung und Coprozessierung eines Supersprengmittels mit Cellulosepulver erreicht, wobei das Cellulosepulver wie ein Docht die Feuchtigkeit aufnimmt, die das Supersprengmittel zum Quellen und Sprengen benötigt. Die schnelle und starke Quellung der Supersprengmittel wird durch eine möglichst hohe bzw. optimale Verdichtung des Zweikomponentensystems bei einem trockenen oder feuchten Coprozeß noch zusätzlich erheblich verbessert. Die neuen Produkte sind außerdem durch eine sehr gute plastische Verformbarkeit und der hiermit verbundenen hervorragenden Tablettierbarkeit charakterisiert. Sie können daher mit Erfolg ohne weitere Zusätze als Tablettensprengmittel (Zerfallsbeschleuniger, Zerfallshilfsmittel) eingesetzt werden.

Als unlösliche Quellmittel und Tablettensprengmittel (Zerfallsbeschleuniger, Zerfallhilfsmittel) werden Hilfsstoffe bezeichnet, die für den raschen Zerfall von Tabletten in Wasser oder Magensaft und für die Freisetzung der Pharmaka in fein dispergierter, gut resorbierbarer Form sorgen. Je nach Wirkungsmechanismus handelt es sich um Substanzen, die die Porosität der Komprimate erhöhen und ein großes Adsorptionsvermögen für Wasser besitzen. Beispiele für bekannte unlösliche Quellmittel und Tablettensprengmittel sind Stärke, pulverförmige und mikrokristalline Cellulosen, Cellulosederivate, Alginsäuren, Dextrane und gasentwickelnde Substanzgemische, z.B. Natriumhydrogencarbonat und Zitronen- oder Weinsäure (Brausemischungen) .

Die im Stand der Technik bekannten Tablettensprengmittel auf Basis von Stärke und Cellulosederivaten wirken nicht absolut gleichartig und einheitlich. Sie sind deshalb noch mit folgenden Nachteilen behaftet:

Stärkekörner allein sowie einige synthetische Quellmittel sind elastisch und deshalb schlecht plastisch verformbar bzw. schlecht tablettierbar und deshalb vorwiegend bessere Zerfallsbeschleuniger. Celluloseprodukte sind dagegen mehr plastisch und deshalb unter Druck besser plastisch verformbar bzw. besser tablettierbar. Cellulosetabletten quellen zwar sehr gut, saugen wie ein Docht wäßrige Flüssigkeiten in großer Menge an, ohne jedoch dabei zu zerfallen. Mischungen aus diesen beiden Substanzklassen vereinigen die Vorteile der guten Tablettierbarkeit, der starken Quellung bzw. der schnellen Zerfallbarkeit.

Sehr stark quellende unlösliche Carboxymethylcellulosen, z.B. vernetzte oder niedrig substituierte Carboxymethylcellulosen, sowie deren Calciumsalze werden auch als Supersprengmittel bezeichnet.

Der Erfindung liegt die Aufgabe zugrunde, die Nachteile der bekannten Supersprengmittel zu überwinden und Tablettensprengmittel freizustellen, die beim Einbringen in Wasser sofort, also in Sekundenschnelle, quellen, zerfallen und sich dispergieren. Daneben sollen je nach dem Vermahlungsgrad instantartig gröbere, feine und feinste bis kolloidartige Dispersionen herstellbar sein. Weiterhin sollen erfindungsgemäß Dispersions- bzw. Suspensionsstabilisatoren bereitgestellt werden, die mit Vorteil bei der Herstellung von Pharmazeutika, Kosmetika, Nahrungsmitteln, die sich sehr rasch dispergieren sollen, verwendet werden können.

Diese Aufgabe wird erfindungsgemäß durch ein Polysaccharidprodukt, bestehend aus in wasser unlösliche, stark quellende Carboxymethylcellulose und pulverisierte oder mikrokristalline Cellulose (MCC) gemäß Anspruch 1, gelöst.

Eine bevorzugte Ausführungsform der Erfindung ist dadurch gekennzeichnet, daß die Carboxymethylcellulose Calcium- oder Natrium-Carboxymethylcellulose ist.

Erfindungsgemäß werden die bekannten Supersprengmittel des Typs der in Wasser unlöslichen Calcium- oder Natrium-Carboxymethylcellulosen durch Kombination mit leicht plastisch verformbaren und gut Flüssigkeit aufsaugenden pulverförmigen oder mikrokristallinen Cellulosen verbessert.

Erfindungsgemäß können alle beliebigen im Handel erhältlichen in Wasser unlöslichen Carboxymethylcellulosen (internationale nicht schützbare Kurzbezeichnung = Croscarmellose), insbesondere die in Wasser unlöslichen Calcium- oder Natrium-Carboxymethylcellulosesorten verwendet werden. Geeignete unlösliche Carboxymethylcelluloseprodukte werden beispielsweise in den Firmenprospekten über vernetzte oder niedrigsubstituierte Natrium- oder Calcium-Carboxymethylcellulose, beispielsweise die Typen Nymcel^{®} R ZSB 10, ZSX, ZSC, ZSB 16) der Firmen METSA Nijmegen, The Netherlands und Ac-Di-Sol^{®} der FMC Corp., Philadelphia, PA 19103, USA, beschrieben.

Bevorzugte in Wasser unlösliche Carboxymethylcelluloseprodukte sind handelsübliche hochmolekulare, unlösliche niedrigsubstituierte oder vernetzte Carboxymethylcellulosen in Form der Calcium- oder Natriumsalze.

Das erfindungsgemäße, durch spezielle Coprozessierung von in Wasser unlöslicher Carboxymethylcellulose mit pulverisierter oder mikrokristalliner Cellulose (MCC) erhaltene Polysaccharidprodukt ist durch folgende Kenndaten charakterisiert:

Bei den gemahlenen, coprozessierten Zubereitungen sollen die Schüttdichten der Produkte mit Cellulosepulver bei 0,120-0,600 g/ml liegen, vorzugsweise bei 0,250-0,500 g/ml. Bei den speziellen Coprodukten mit mikrokristalliner Cellulose (MCC) und in Wasser unlöslicher Carboxymethylcellulose liegen die Schüttdichten bei 0,300-0,750 g/ml, vorzugsweise bei 0,350-0,600 g/ml. Die Schüttdichte des erfindungsgemäßen Polysaccharid-Produkts ist vorzugsweise höher als die Schüttdichte des Ausgangspulvergemisches.

Insbesondere ist das Produkt durch einen Restfeuchtegehalt von 1,5 bis 15%, vorzugsweise von weniger als 10%, beispielsweise 4 bis 7%, charakterisiert.

Der Gehalt der einzelnen Komponenten kann innerhalb weiter Grenzen variieren, beispielsweise von 1 bis 60 Gew.-% in Wasser unlöslicher Carboxymethylcellulose und 40 bis 99 Gew.-% pulverisierter oder mikrokristalliner Cellulose. Bevorzugt wird ein Gehalt von 3 bis 60 Gew.-% in Wasser unlöslicher Carboxymethylcellulose und 40 bis 97 Gew.-% pulverisierter oder mikrokristalliner Cellulose. Ganz bevorzugt werden Mengen von 5 bis 30 Gew.-% in Wasser unlöslicher Carboxymethylcellulose und 70 bis 95 Gew.-% pulverisierter oder mikrokristalliner Cellulose.

Auch die Korngröße des erfindungsgemäßen coprozessierten Polysaccharidprodukts kann innerhalb weiter Grenzen variieren, wie beispielsweise von 10 bis 2000 µm, vorzugsweise 50 bis 1000 µm.

Das erfindungsgemäße Polysaccharidprodukt kann durch eine spezielle Coprozessierung bzw. gemeinsame Behandlung von in Wasser unlöslicher Carboxymethylcellulose mit pulverisierter oder mikrokristalliner Cellulose durch feuchte oder trockene Aggregierung unter Druck erhalten werden. Unter dem Begriff "Coprozessierung" wird hier eine trockene Kompaktierung/Aggregierung, z.B. zwischen gegenläufigen Kompaktierwalzen bei Drücken von 20-60 kN, vorzugsweise 30-50 kN, oder eine feuchte Kompaktierung/Aggregierung nach Zusatz von Wasser, durch Kneten oder Pressen von feuchtplastischen Massen durch ein Sieb, eine Lochscheibe oder über einen Extruder und abschließendem Trocknen verstanden.

Durch die Erfindung wird weiterhin ein Verfahren zur Herstellung des oben beschriebenen Polysaccharidprodukts bereitgestellt, welches die Coprozessierung von in Wasser unlöslicher Carboxymethylcellulose mit mikrokristalliner oder pulverisierter Cellulose umfaßt.

Eine Alternative des erfindungsgemäßen Verfahrens ist durch folgende Stufen charakterisiert:
(a) Suspendieren und gegebenenfalls Verdichten von feuchter bzw. nicht getrockneter, nicht verhornter mikrokristalliner oder pulverisierter Cellulose in einem, vorzugsweise heißen, wäßrigen Medium zu einer homogenen Dispersion;
(b) Vermengen der in Stufe (a) erhaltenen Suspension oder pastenförmigen vorverdichteten Massen nach Abkühlen unter 40°C mit in Wasser unlöslicher Carboxymethylcellulose;
(c) gegebenenfalls erfolgendes Vortrocknen;
(d) Aggregieren unter Druck, insbesondere verdichtendes Passieren, vorzugsweise Pressen der in Stufe (b) bzw. (c) erhaltenen und vorverdichteten feuchtplastischen Masse durch ein Sieb, eine Lochscheibe oder einen Extruder;
(e) Trocknen der in Stufe (d) erhaltenen aggregierten Masse bei einer Temperatur von 20 bis 90°C; und
(f) Vermahlen bzw. Brechen des so erhaltenen Produkts auf eine mittlere Korngröße von 10 bis 2000 µm, vorzugsweise 10-1000 µm.

In der ersten Stufe dieses Verfahrens wird feuchte, nicht verhornte pulverförmige oder mikrokristalline Cellulose in einem wäßrigen Medium zu einer homogenen Dispersion suspendiert.

Als Cellulose-Ausgangsprodukt wird vorzugsweise ein nicht getrocknetes mikrokristallines Celluloseprodukt oder pulverisierte Cellulose verwendet. Die pulverförmige Cellulose wird beispielsweise in an sich bekannter Weise aus Zellstoff oder faseriger Rohcellulose hergestellt. Die mikrokristalline Cellulose wird aus Zellstoffpulpe durch Erhitzen mit einer 3- bis 5%-igen starken Mineralsäure, beispielsweise Salz- oder Schwefelsäure, hergestellt. Für die Zwecke der Erfindung besonders geeignete, nicht getrockneten Celluloseprodukte sind praktisch Zwischen- oder Vorprodukte der Herstellung von reiner Pulvercellulose oder mikrokristalliner Cellulose.

Die oben erwähnte Herstellung von mikrokristalliner Cellulose aus Zellstoff oder von Pulvercellulose wird beispielsweise von B. Philipp und H.H. Steege in "Wissenschaft und Fortschritt" 25 (3), 126-131 (1975) beschrieben.

Unter "nicht getrocknet" werden Cellulosevorprodukte verstanden, die aus dem Herstellungsgang, nach der Reinigung, aber vor der Endtrocknung gewonnen werden, z.B. bei der oben beschriebenen Behandlung von Zellstoffpulpe mit Mineralsäure nach Entfernen der Säure und der erforderlichen Reinigung der hierbei entstandenen mikrokristallinen Cellulose. Das Cellulose:Wasser-Verhältnis kann 1,0:0,15 Teile bis 1,0:30,0 Teile, vorzugsweise 1,0:1,0 Teile bis 1,0:10,0 Teile betragen. Bei einer Trocknung des Produkts zu einem Feuchtigkeitsgehalt von weniger als 1,5 Teile Wasser pro 10,0 Teile Cellulose kann es vorkommen, daß die Oberfläche der Cellulose zu trocken wird und daß das Produkt in unerwünschter Weise "verhornt". In solchen Fällen sind die Polysaccharidprodukte nicht mehr optimal für die oben angegebenen Zwecke geeignet, da verhornte Bereiche nur mehr stark eingeschränkt hydratisieren können. Andererseits führen zu große Flüssigkeits- bzw. Wassermengen dazu, daß die Produkte nach der Zugabe von in Wasser unlöslicher Carboxymethylcellulose nicht ausreichend feuchtplastisch werden, sondern zu flüssig sind, so daß eine Obergrenze von 30,0 Teile, vorzugsweise 25,0 Teile Wasser pro 1,0 Teil Cellulose bevorzugt wird. Außerdem ist das Trocknen von hohen Feuchtigkeitsanteilen nicht wirtschaftlich. Relativ hohe Mengen von Wasser sind zu einer ausreichenden Verdichtung erforderlich, aber zu hohe Mengen können entsprechend den in Stufe (b) verwendeten Mengen der in Wasser unlöslichen Carboxymethylcellulose und dem Polymerisationsgrad dieses Produkts die Gemische dann zu feucht werden lassen, so daß sie nicht mehr feuchtplastisch verformbar bzw. aggregierbar sind und nur noch langwierig unter Schwierigkeiten getrocknet werden können.

Somit wird in der ersten Stufe dieses Verfahrens die oben beschriebene nicht verhornte Cellulose in einem wäßrigen, zur besseren Hydratisierung vorzugsweise warmen bis heißen Medium zu einer homogenen Dispersion suspendiert. Als wäßriges Medium kann beispielsweise Wasser, wie Leitungswasser, verwendet werden, das gegebenenfalls bis zum Sieden aufgeheizt werden kann. Die Temperatur beim Suspendierungsvorgang beträgt 40 bis 100°C, vorzugsweise 80 bis 100°C. Die Suspendierung kann in an sich bekannter Weise in einer geeigneten Vorrichtung, wie einem Schnellmischer oder Dispergator, verwendet werden. Geeignete Vorrichtungen sind im Handel erhältlich und werden beispielsweise in Bauer, Frömming, Führer: Pharmazeutische Technologie, G. Fischer Verlag, Stuttgart, Jena, 5. Auflage, 1997, Seite 135 beschrieben. Bei dieser Befeuchtung, d.h. bei diesem Suspensionsvorgang, kann gegebenenfalls schon eine Vorverdichtung erzielt werden, z.B. durch stärkere Hydratation bei Verwendung von heißem Wasser. Weiterhin kann durch Kneten, beispielsweise im Z-Kneter mit kräftigen Mischarmen, im Gegensatz zu einfach gerührten bzw. gemischten Massen eine stärkere Verdichtung oder Vorverdichtung erreicht werden. Die so erhaltene Dispersion hat beispielsweise einen Feststoffgehalt von 10 bis 90 Gew.-%, vorzugsweise 20 bis 60 Gew.-%.

In der nächsten Stufe (b) dieses Verfahrens wird die in Stufe (a) erhaltene Suspension nach Abkühlung unter 40°C mit in Wasser unlöslicher Carboxymethylcellulose vermengt.

Die Menge der in dieser Stufe zugegebenen in Wasser unlöslichen Carboxymethylcellulose wird so bemessen, daß die als Endprodukt erhaltenen Polysaccharidprodukte 3,0 bis 60 Gew.-%, vorzugsweise 5 bis 40 Gew.-%, in Wasser unlösliche Carboxymethylcellulose enthalten.

Die Vermengung der beiden Ausgangsprodukte kann vorzugsweise durch Verkneten geschehen. Geeignete Kneter werden beispielsweise in Bauer, Frömming, Führer: Pharmazeutische Technologie, G. Fischer Verlag, Stuttgart, Jena, 5. Auflage, 1997, Seite 134-135 beschrieben. Die Temperatur bei der Vermengung bzw. Verknetung beträgt 50 bis 30°C, vorzugsweise 40 bis 20°C. Vorzugsweise erfolgt die Verknetung bei Raumtemperatur, damit das Stärkeprodukt nicht nachteilig beeinflußt wird. Eine Verknetung kann daneben auch grundsätzlich dann vorgenommen werden, um eine Verdichtung zu erzielen.

In der nächsten Stufe (c) dieses Verfahrens wird das in Stufe (b) erhaltene Gemisch gegebenenfalls zu einem Restfeuchtegehalt von 1,5 bis 15 Gew.-%, vorzugsweise 4 bis 7 Gew.-%, vorgetrocknet. Als Trockner können übliche Trocknungsvorrichtungen, wie Trockenschränke und Wirbelschichttrockner eingesetzt werden. Geeignete Trockner werden beispielsweise in Bauer, Frömming, Führer: Pharmazeutische Technologie, G. Fischer Verlag, Stuttgart, Jena, 5. Auflage, 1997, Seiten 123-130 beschrieben. Bevorzugt wird die Vortrocknung bei Temperaturen von 30 bis 70°C, insbesondere 50 bis 60°C durchgeführt.

In der nächsten Stufe (d) dieses Verfahrens wird das in Stufe (b) bzw. (c) erhaltene feuchtplastische, durch das Befeuchten bereits vorverdichtete Produkt durch ein Sieb geschlagen bzw. gepreßt und dadurch möglichst weitgehend verdichtet. Als Siebe kommen beispielsweise übliche Siebe, die beispielsweise in Bauer, Frömming, Führer: Pharmazeutische Technologie, G. Fischer Verlag, Stuttgart, Jena, 5. Auflage, 1997, Seite 108 (Siebe), Seite 308 (Extrudergranulierung) beschrieben werden, in Betracht. Geeignete Siebe haben eine Maschenweite von 0,5 mm bis 5 mm, vorzugsweise 1-2 mm. Im übrigen erfolgt das Durchpressen bzw. -schlagen des Produkts durch Siebe bzw. durch Extruder in üblicher Weise und bei üblichen Bedingungen, wie Normaltemperatur. Das Sieben erfolgt bei Normaldruck oder gegebenenfalls unter Anwendung von Überdruck.

In der nächsten Stufe (e) dieses erfindungsgemäßen Verfahrens wird die durch das Sieb geleitete Masse bei einer Temperatur von 20 bis 90°C, vorzugsweise 50 bis 80°C getrocknet. Die Trocknung erfolgt in der gleichen Weise wie die oben im Zusammenhang mit Stufe (c) beschriebene Vortrocknung. Auch hier können übliche Trockner, wie Trockenschränke und Wirbelschichttrockner, eingesetzt werden.

In der letzten Stufe (f) dieses Verfahrens wird schließlich das so erhaltene Produkt auf eine mittlere Korngröße von 10 bis 1000 µm, vorzugsweise 50 bis 500 µm, vermahlt. Für den Vermahlungsvorgang können alle beliebigen geeigneten Mühlen, wie beispielsweise Kugelmühlen und dergleichen, verwendet werden. Geeignete Vorrichtungen sind beispielsweise in Bauer, Frömming, Führer: Pharmazeutische Technologie, G. Fischer Verlag, Stuttgart, Jena, 5. Auflage, 1997, Seite 104-107 beschrieben.

Eine andere Alternative des erfindungsgemäßen Verfahrens ist durch folgende Stufen charakterisiert:
(a) Vermischen der pulverförmigen Bestandteile in Wasser unlösliche Carboxymethylcellulose und Cellulose;
(b) Sieben durch ein feines Sieb;
(c) Verdichten durch Kompaktieren oder Brikettieren mit Kompaktierwalzen oder einer Exzenterpresse bei Drücken von 20-60 kN, vorzugsweise 30-50 kN;
(d) Granulieren bzw. verkleinerndes Homogenisieren der nach (c) erhaltenen Briketts oder Schülpen mit Stachelwalzen oder einem ähnlichen Gerät oder Vermahlen mit einer geeigneten Mühle auf Korngrößen von 10-2000 µm ; und
(e) Vermahlen des so erhaltenen Produkts auf eine mittlere Korngröße von 10 bis 1000 µm.

Bei dieser Alternative entfallen die Herstellungsschritte der Suspendierung (a), des Vortrocknens (c), das Pressen durch Siebe oder Extruder (d) und der Endtrocknung (e) der ersten Alternative. Es verbleiben nur die Schritte Vermischen der beiden Komponenten (a), einfaches Sieben (b), zum Beispiel durch ein Sieb mit einer Maschenweite von 1,2 mm, und als Hauptschritt die Kompaktierung mit Druck- bzw. Kalanderwalzen oder Exzenterpressen (c), die nachfolgende Zerkleinerung der kompaktierten Schülpen oder Briketts mit Stachelwalzen (d) und das Vermahlen mit geeigneten Mühlen (e).

Die Qualität der Verdichtung hängt von den Kompaktierdrucken (20-60 kN, vorzugsweise 30-50 kN) und in zweiter Linie auch noch von den Restfeuchtigkeitsgehalten der Mischung (b) ab, die zwischen 3 und 40 Gew.-%, vorzugsweise zwischen 5 und 25 Gew.-% liegen kann. Endprodukte mit den höheren Feuchtigkeitsgehalten müssen evtl. nachgetrocknet werden, bis sie Restfeuchtigkeiten von 1,5-15 Gew.-%, vorzugsweise von 4-7 Gew.-% aufweisen. Die Trockenverdichtungs- bzw. Trockengranulierverfahren werden beispielsweise in Bauer, Frömming, Führer: Pharmazeutische Technologie, G. Fischer Verlag, Stuttgart, Jena, 5. Auflage, 1997, Seite 306, 308 und 310 beschrieben.

Die so hergestellten erfindungsgemäßen, mit in Wasser unlöslicher Carboxymethylcellulose coprozessierten Celluloseprodukte zeichnen sich durch eine außerordentlich schnelle und starke Quellung beim Einbringen in Wasser oder wasserhaltige Flüssigkeiten sowie durch eine sehr gute Tablettierbarkeit aus. Sie können daher als äußerst wirksame Tablettensprengmittel (Zerfallhilfsmittel bzw. Zerfallsbeschleuniger) eingesetzt werden.

Charakteristisch für die erfindungsgemäßen hochwirksamen Sprengmittel ist der durch die innige Mischung und Coprozessierung des Supersprengmittels mit der Cellulose erreichte enge Kontakt dieser beiden Hilfsstoffe. Dieser enge Kontakt soll auch nach dem Einarbeiten von Wirkstoffen (z.B. Arzneimittel o.a.) und der anderen Komponenten in die Tablettenrezeptur erhalten bleiben. Denn dieser enge Kontakt ist für die optimale Sprengwirkung entscheidend.

Ein weiterer Vorteil dieser erfindungsgemäß intensiv coprozessierten und kombinierten Zufallsbeschleuniger ist es, daß auch mechanisch feste, stark komprimierte, wenig poröse Tabletten hergestellt werden können.

Unter Verwendung der erfindungsgemäßen Produkte können Dispersionstabletten hergestellt werden, die beim Einbringen in Wasser sofort, also in Sekundenschnelle, quellen, zerfallen und sich dispergieren. Je nach Vermahlungsgrad des erfindungsgemäßen Produkts lassen sich aus den damit hergestellten Tabletten instantartig gröbere, feine und feinste bis kolloidartige Dispersionen erzeugen. Neben der Verwendbarkeit als Tablettenzerfallsbeschleuniger ist natürlich ein Einsatz als Dispersions- oder Suspensionsstabilisator bei der direkten Herstellung von flüssigen und halbfesten Arzneizubereitungen wie Sirupen, Lotionen, Säften, Gelen, Hydrosolen und Hydrogelen, Salbenzubereitungen und Pasten denkbar und möglich. Die Einsatzmöglichkeiten sind überall möglich, wo schnelle und feinste Dispergierungen erreicht und stabilisiert werden sollen. Die Anwendung ist nicht auf Pharmazeutika und Kosmetika beschränkt. Es drängen sich auch Verwendungen bei nahrungsmitteltechnologischen und technischen Aufgabenstellungen auf. Beispielsweise soll hier die Stabilisierung von Speiseeis oder Softeis, die Verdickung und Dispergierung von Soßen genannt werden, sowie auch Instantzubereitungen, wie Kakao- und andere Mixgetränke, die aus pulver- oder granulatartigen Vorprodukten augenblicklich anrührbar oder dispergierbar sein sollen. Des weiteren Waschmittel-, Reinigungsmittel-, Entkalkungstabletten, die schnell im Wasch- oder Spülmittelschacht zerfallen sollen.

Die Erfindung wird in den Beispielen erläutert.

### 1. Herstellungsbeispiele

### Beispiel 1:

1000,0 kg feuchtes, nicht verhorntes Cellulosepulver mit einem Wassergehalt von ca. 20% wird möglichst bald nach der Herstellung durch Zugabe von 2500,0 kg Wasser resuspendiert. Die Suspension wird mit einem Schnellmischer oder Dispergator homogen dispergiert, und anschließend werden 160,0 kg handelsüblicher unlöslicher Carboxymethylcellulose (beispielsweise Ac-Di-Sol^{®}) eingeknetet. Die feuchtplastische Masse wird durch einen Extruder mit 1 mm Maschenweite geschlagen und in einem geeigneten Trockner bei 60°C getrocknet. Das fertig coprozessierte Produkt hat einen Restfeuchtegehalt von 5-8 Gew.-% und mittlere Partikelgrößen von 0,25-0,8 mm.

### Beispiel 2:

2250,0 kg feuchter Kuchen mit einem Verhältnis mikrokristalline Cellulose:Wasser = 40:60 wird mit der gleichen Menge Wasser resuspendiert und homogenisiert. Anschließend werden 100,0 kg unlösliche Carboxymethylcellulose z.B. Calcium-Carboxymethylcellulose (beispielsweise Nymcel^{®} ZSB 16), gleichmäßig eingeknetet, die feuchtplastische Masse durch ein 1,5 mm Sieb geschlagen und bei 80°C in einem Wirbelschichttrockner bis zu einer Restfeuchte von ca. 5% getrocknet.

### Beispiel 3:

2000,0 kg feuchter Kuchen aus mikrokristalliner Cellulose und Wasser im Verhältnis 50:50 wird mit der doppelten Menge Wasser angeschlämmt bzw. resuspendiert und 5 min lang mit dem Ultra-Turrax (Rotor-Stator-Mixer) homogenisiert.

Anschließend wird die homogene Mischung in einen Planetenmischen gegeben, und bei laufendem Rührwerk wird portionsweise 300,0 kg unlösliche Carboxymethylcellulose (beispielsweise Nymcel^{®} ZSX) dazugeknetet und abschließend 10 min gemischt.

Die feuchtplastische Masse wird in dünnen Schichten und angemessenen Portionen entweder im Trockenschrank bei 50°C oder im Wirbelschicht-Trockner (Zulufttemperatur 70°C-120°C) bis zur siebbaren Konsistenz vorgetrocknet. Dann wird sie durch ein 5mm-Sieb geschlagen und im Wirbelschicht-Trockner bei einer Zulufttemperatur von 70°-80°C zu Ende getrocknet (Restfeuchte ca. 5%).

Zuletzt wird die Masse durch ein 1,00 mm-Sieb geschlagen und in einer geeigneten Mühle auf eine mittlere Korngröße von 80 µm gemahlen.

### Beispiel 4:

| | |
|---|---|
| 2000,0 g | feuchtes, feingemahlenes Cellulosepulver (mittlere Partikelgröße 50 µm, Feuchtigkeitsgehalt ca. 50%) wird mit |
| 1500,0 g | siedendem, deionisiertem Wasser in einem geeigneten Mischer homogen befeuchtet. Anschließend wird |
| 100,0 g | unlösliche Carboxymethylcellulose (beispielsweise Nymcel^{®}ZSC) eingeknetet, so daß eine feuchtplastische Masse entstanden ist. Die feuchtplastische Masse wird mit möglichst hohem Druck durch einen Extruder mit einer Lochscheibe mit 1-3 mm-Bohrungen (vorzugsweise 1-2 mm) gepreßt. Das entstandene Extrudergranulat wird bei 80°C bis zu einer Restfeuchte von 4-6% getrocknet und anschließend mit einer Rotor-Stator- oder Stift-Mühle auf eine mittlere Korngröße von 0,2 mm vermahlen. |

### Beispiel 5:

| | |
|---|---|
| 9,0 kg | feingemahlenes Cellulosepulver (mittlere Partikelgröße 70-80 µm) und |
| 1,0 kg | unlösliche Carboxymethylcellulose (beispielsweise Nymcel^{®} ZSB 10) werden homogen gemischt und durch ein 1 mm-Sieb geschlagen. Anschließend wird diese Mischung mit Hilfe einer Kompaktierwalze bei Drucken von 30-45 kN brikettiert und ein Korngrößenbereich von 0,2 mm bis 1 mm ausgesiebt. Die Schüttdichte des so hergestellten Produkts liegt bei 0,400-0,450 g/ml. |

### 2. Verwendungsbeispiel

Unter Verwendung der erfindungsgemäßen, mit unlöslicher Carboxymethylcellulose coprozessierten gemahlenen Cellulose wurden Tabletten in folgender Weise hergestellt:

| | |
|---|---|
| ASS (Acetylsalicylsäure), mittlere Korngröße 0,2 mm | 1000,0 |
| Cellulose- unlösliche Carboxymethylcellulose Coprocessed | 100,0 |

werden gemischt und auf einer üblichen Tablettenpresse zu Tabletten mit 11 mm Durchmesser und 550 mg Bruttogewicht verpreßt.

Die im Verwendungsbeispiel hergestellten Tabletten wurden folgendem Zerfallstest in folgender Weise unterworfen:
a) einfaches Einlegen der Tabletten in ein Becherglas mit ca. 200-250 ml reines Wasser bei Raumtemperatur und Beobachtung des Zerfalls;
b) Einbringen jeweils einer Tablette, die in einem Drahtbügel eingeklemmt ist, in ein Becherglas mit 250 ml reinem Wasser bei Raumtemperatur und Bestimmung des Zeitraums bis zum vollständigen Zerfall.

Die erhaltenen Ergebnisse sind in folgender Tabelle zusammengestellt:

| | a) | b) |
|---|---|---|
| Tabl. mit 500 mg ASS + 50 mg Cellulose coprozessiert nach Beispiel 1: | zerfällt sehr rasch | 10-15 s |
| Tabl. mit 500 mg ASS + 50 mg Cellulose coprozessiert nach Beispiel 2: | " | 20-25 s |
| Tabl. mit 500 mg ASS + 50 mg Cellulose coprozessiert nach Beispiel 5: | " | 16-17 s |
| Tabl. mit 500 mg ASS + 50 mg Weizenstärke: | quillt stark, aber zerfällt nicht | 35-45 s |

Aus der obigen Tabelle ergibt sich, daß die unter Verwendung der erfindungsgemäßen Produkte hergestellten Tabletten im Vergleich zu den Produkten mit herkömmlichen Sprengmitteln wesentlich verbesserte Zerfallseigenschaften haben.

## Patentansprüche

1. Polysaccharidprodukt, bestehend aus in Wasser unlöslicher Carboxymethylcellulose und mikrokristalliner oder pulverisierter Cellulose **dadurch gekennzeichnet, dass** die in Wasser unlösliche Carboxymethylcellulose und die mikrokristalline oder pulverisierte Cellulose durch Coprozessierung mittels trockener Aggregierung bei Drücken von 20-60 kN oder feuchte Aggregierung nach Zusatz von Wasser, durch Kneten oder Pressen von feuchtplastischen Massen durch ein Sieb, eine Lochscheibe oder über einen Extruder und anschließendem Trocknen unter Druck zusammengebracht und verdichtet sind.

2. Polysaccharidprodukt nach Anspruch 1, **dadurch gekennzeichnet, daß** die in Wasser unlösliche Carboxymethylcellulose in Wasser unlösliche Calcium- oder NatriumCarboxymethylcellulose ist.

3. Polysaccharidprodukt nach Anspruch 1 oder 2, **dadurch gekennzeichnet, daß** es eine Restfeuchte von weniger als 10% besitzt.

4. Polysaccharidprodukt nach einem der Ansprüche 1 bis 3, **dadurch gekennzeichnet, daß** es 3 bis 60 Gew.-% in Wasser unlösliche Carboxymethylcellulose und 40 bis 97 Gew.-% mikrokristalline oder pulverisierte Cellulose enthält.

5. Polysaccharidprodukt nach einem der Ansprüche 1 bis 4, **dadurch gekennzeichnet, daß** es eine mittlere Korngröße von 10 bis 2000 µm, vorzugsweise 10-1000 µm, hat.

6. Polysaccharidprodukt, nach einem der Ansprüche 1 bis 5, **dadurch gekennzeichnet, dass** es gegenüber dem Ausgangspulvergemisch eine höhere Schüttdichte hat.

7. Verfahren zur Herstellung eines Polysaccharidprodukts, gemäß den Ansprüchen 1 bis 6 umfassend die Coprozessierung mittels trockener Aggregierung bei Drücken von 20-60 kN oder feuchte Aggregierung nach Zusatz von Wasser, durch Kneten oder Pressen von feuchtplastischen Massen durch ein Sieb, eine Lochscheibe oder über einen Extruder und anschließendem Trocknen unter Druck von in Wasser unlöslicher Carboxymethylcellulose mit mikrokristalliner oder pulverisierter Cellulose.

8. Verfahren nach Anspruch 7, **dadurch gekennzeichnet, daß** es die folgenden Stufen umfaßt:
(a) Suspendieren und gegebenenfalls Verdichten von feuchter bzw. nicht getrockneter, nicht verhornter mikrokristalliner oder pulverisierter Cellulose in einem wäßrigen Medium zu einer homogenen Dispersion;
(b) Vermengen der in Stufe (a) erhaltenen Suspension oder pastenförmigen, vorverdichteten Massen nach Abkühlen unter 40°C mit in Wasser unlöslicher Carboxymethylcellulose;
(c) gegebenenfalls erfolgendes Vortrocknen;
(d) Verdichtendes Passieren, vorzugsweise Pressen der in Stufe (b) bzw. (c) erhaltenen und vorverdichteten feuchtplastischen Masse durch ein Sieb, eine Lochscheibe oder einen Extruder;
(e) Trocknen der in Stufe (d) erhaltenen aggregierten Masse bei einer Temperatur von 20 bis 90°C; und
(f) Vermahlen des so erhaltenen Produkts auf eine mittlere Korngröße von 10 bis 2000 µm vorzugsweise 10-1000 µm.

9. Verfahren nach Anspruch 8, **dadurch gekennzeichnet, daß** das Vermengen und Verdichten durch Verkneten erfolgt.

10. Verfahren nach Anspruch 7, **dadurch gekennzeichnet, daß** es die folgenden Stufen umfaßt:
(a) Vermischen der pulverförmigen Bestandteile in Wasser unlösliche Carboxymethylcellulose und Cellulose;
(b) Sieben durch ein feines Sieb;
(c) Verdichten durch Kompaktieren oder Brikettieren mit Kompaktierwalzen oder einer Exzenterpresse bei Drücken von 20-60 kN, vorzugsweise 30-50 kN;
(d) Granulieren bzw. verkleinerndes Homogenisieren der nach (c) erhaltenen Briketts oder Schülpen mit Stachelwalzen oder einem ähnlichen Gerät oder Vermahlen mit einer geeigneten Mühle auf Korngrößen von 10-2000 µm; und
(e) Vermahlen des so erhaltenen Produkts auf eine mittlere Korngröße von 10 bis 1000 µm.

11. Verfahren nach einem der Ansprüche 7 bis 10, **dadurch gekennzeichnet, daß** die in Wasser unlösliche Carboxymethylcellulose in Wasser unlösliche Calcium- oder Natrium-Carboxymethylcellulose ist.

12. Verwendung des Polysaccharidprodukts nach den Ansprüchen 1 bis 6 als Dispersions- oder Suspensionsstabilisator bei der Herstellung von flüssigen und halbfesten Zubereitungen.

13. Verwendung des Polysaccharidprodukts nach den Ansprüchen 1 bis 6 als Tablettensprengmittel.

## Claims

1. Polysaccharide product consisting of water-insoluble carboxymethylcellulose and microcrystalline or pulverized cellulose, **characterized in that** the water-insoluble carboxymethylcellulose and the microcrystalline or pulverized cellulose are combined and compressed by coprocessing by means of dry aggregation at pressures of 20-60 kN or moist aggregation after addition of water, by kneading or pressing wet-plastic materials through a screen, a perforated disc or by means of an extruder, and subsequently drying under pressure.

2. Polysaccharide product according to Claim 1, **characterized in that** the water-insoluble carboxymethylcellulose is water-insoluble calcium carboxymethylcellulose or sodium carboxymethylcellulose.

3. Polysaccharide product according to Claim 1 or 2, **characterized in that** it has a residual moisture content of less than 10%.

4. Polysaccharide product according to one of Claims 1 to 3, **characterized in that** it contains 3 to 60% by weight of water-insoluble carboxymethylcellulose and 40 to 97% by weight of microcrystalline or pulverized cellulose.

5. Polysaccharide product according to one of Claims 1 to 4, **characterized in that** it has a mean particle size of 10 to 2000 µm, preferably 10-1000 µm.

6. Polysaccharide product according to one of Claims 1 to 5, **characterized in that** it has a higher bulk density than the starting powder mixture.

7. Process for preparing a polysaccharide product according to Claims 1 to 6, comprising coprocessing by means of dry aggregation at pressures of 20-60 kN or moist aggregation after addition of water, by kneading or pressing wet-plastic materials through a screen, a perforated disc or by means of an extruder, and subsequent drying under pressure, of water-insoluble carboxymethylcellulose with microcrystalline or pulverized cellulose.

8. Process according to Claim 7, **characterized in that** it comprises the following stages:
(a) suspending and optionally compacting moist or undried, unhornified microcrystalline or pulverized cellulose in an aqueous medium to give a homogeneous dispersion;
(b) mixing the suspension or pasty precompacted materials obtained in stage (a), after cooling below 40°C, with water-insoluble carboxymethylcellulose;
(c) optionally predrying;
(d) compressive conveying, preferably pressing, of the precompacted wet-plastic material obtained in stage (b) or (c) through a screen, a perforated disc or an extruder;
(e) drying the aggregated material obtained in stage (d) at a temperature of 20 to 90°C; and
(f) grinding the product thus obtained to a mean particle size of 10 to 2000 µm, preferably 10-1000 µm.

9. Process according to Claim 8, **characterized in that** the mixing and compaction are effected by kneading.

10. Process according to Claim 7, **characterized in that** it comprises the following stages:
(a) mixing the pulverulent constituents water-insoluble carboxymethylcellulose and cellulose;
(b) screening through a fine screen;
(c) compression by compacting or briquetting with compacting rolls or an eccentric press at pressures of 20-60 kN, preferably 30-50 kN;
(d) granulating or size-reducing homogenizing of the briquettes or slugs obtained in (c) with spiked rolls or a similar unit or grinding with a suitable mill to particle sizes of 10-2000 µm; and
(e) grinding the product thus obtained to a mean particle size of 10 to 1000 µm.

11. Process according to one of Claims 7 to 10, **characterized in that** the water-insoluble carboxymethylcellulose is water-insoluble calcium carboxymethylcellulose or sodium carboxymethylcellulose.

12. Use of the polysaccharide product according to Claims 1 to 6 as a dispersion or suspension stabilizer in the production of liquid and semisolid formulations.

13. Use of the polysaccharide product according to Claims 1 to 6 as a tablet disintegrant.

## Revendications

1. Produit de type polysaccharide, constitué par de la carboxyméthylcellulose insoluble dans l'eau et par de la cellulose microcristalline ou pulvérisée, **caractérisé en ce que** la carboxyméthylcellulose insoluble dans l'eau et **en ce que** la cellulose microcristalline ou pulvérisée sont regroupées et comprimées par un co-traitement au moyen d'une agrégation à l'état sec à une pression de 20 à 60 kN ou d'agrégation à l'état humide après addition d'eau, par un malaxage ou un pressage de masses plastiques humides à travers un crible, un plateau perforé ou par une extrudeuse et par séchage consécutif sous pression.

2. Produit de type polysaccharide selon la revendication 1, **caractérisé en ce que** la carboxyméthylcellulose insoluble dans l'eau est de la carboxyméthylcellulose de calcium ou de sodium insoluble dans l'eau.

3. Produit de type polysaccharide selon la revendication 1 ou 2, **caractérisé en ce qu'**il comprend une humidité résiduelle inférieure à 10 %.

4. Produit de type polysaccharide selon l'une des revendications 1 à 3, **caractérisé en ce qu'**il contient 3 à 60 % en poids de carboxyméthylcellulose insoluble dans l'eau et 40 à 97 % en poids de cellulose microcristalline ou pulvérisée.

5. Produit de type polysaccharide selon l'une des revendications 1 à 4, **caractérisé en ce qu'**il a une granulométrie moyenne de 10 à 2000 µm, de préférence, de 10 à 1000 µm.

6. Produit de type polysaccharide selon l'une des revendications 1 à 5, **caractérisé en ce qu'**il a une plus grande densité apparente comparé au mélange pulvérulent initial.

7. Procédé pour préparer un produit de type polysaccharide selon les revendications 1 à 6, comprenant le co-traitement au moyen d'une agrégation à l'état sec à une pression de 20 à 60 kN ou d'une agrégation à l'état humide après addition d'eau, par un malaxage ou un pressage de masses plastiques humides à travers un crible, un plateau perforé ou par une extrudeuse et par séchage consécutif sous pression d'une carboxyméthylcellulose insoluble dans l'eau avec une cellulose microcristalline ou pulvérisée.

8. Procédé selon la revendication 7, **caractérisé en ce qu'**il comprend les étapes suivantes consistant à :
(a) mettre en suspension et, le cas échéant, comprimer la cellulose microcristalline ou pulvérisée humide ou non séchée, non durcie, dans un milieu aqueux pour obtenir une dispersion homogène ;
(b) mélanger la suspension obtenue à l'étape (a) ou des masses pâteuses préalablement comprimées après refroidissement en dessous de 40 °C avec la carboxyméthylcellulose insoluble dans l'eau ;
(c) procéder le cas échéant à un séchage préalable ;
(d) procéder à un passage à des fins de compression, de préférence, à un pressage de la masse plastique humide obtenue à l'étape (b) ou (c) et préalablement comprimée à travers un crible, un plateau perforé ou une extrudeuse ;
(e) sécher la masse agrégée obtenue à l'étape (d) à une température de 20 à 90 °C ; et
(f) broyer le produit ainsi obtenu selon une granulométrie moyenne de 10 à 2000 µm, de préférence, de 10 à 1000 µm.

9. Procédé selon la revendication 8, **caractérisé en ce qu'**on effectue le mélange et la compression par malaxage.

10. Procédé selon la revendication 7, **caractérisé en ce qu'**il comprend les étapes suivantes consistant à :
(a) mélanger les composants pulvérulents dans la carboxyméthylcellulose insoluble dans l'eau et dans la cellulose ;
(b) procéder à un criblage à travers un crible fin ;
(c) procéder à une compression par compactage ou par briquetage à l'aide de cylindres de compactage ou d'une presse excentrique à une pression de 20 à 60 kN, de préférence, de 30 à 50 kN ;
(d) procéder à une granulation ou à une homogénéisation réductrice de la briquettes ou de la plaquette obtenue selon l'étape (c) avec des cylindres dentés ou un appareil analogue ou bien à un broyage avec un broyeur adapté selon une granulométrie de 10 à 2000 µm ; et
(e) procéder au broyage du produit ainsi obtenu selon une granulométrie moyenne de 10 à 1000 µm.

11. Procédé selon l'une des revendications 7 à 10, **caractérisé en ce que** la carboxyméthylcellulose insoluble dans l'eau est la carboxyméthylcellulose de calcium ou de sodium insoluble dans l'eau.

12. Utilisation du produit de type polysaccharide selon les revendications 1 à 6 en tant que stabilisant pour dispersion ou pour suspension lors de l'élaboration de préparations fluides et semi-solides.

13. Utilisation du produit de type polysaccharide selon les revendications 1 à 6 en tant que désintégrant pour tablettes.
